# EUROPEAN PATENT APPLICATION

(11) **EP 1 932 477 A1**
(43) Date of publication of application: **18.06.2008**
(21) Application number: 06798444.3
(22) Date of filing: 29.09.2006
(51) Int. Cl.: A61B 8/00

(54) **ULTRASONIC PROBE AND ULTRASONIC DIAGNOSTIC DEVICE EMPLOYING SAME**

(30) Priority: 04.10.2005 JP 2005290751
(71) Applicant: Hitachi Medical Corporation, Tokyo 101-0021 (JP)
(72) Inventor: IKEDA, Takashi, Chiyoda-ku, Tokyo 1010021 (JP); OKAZAKI, Hideki, Chiyoda-ku, Tokyo 1010021 (JP); HIRANO, Yoshinori, Chiyoda-ku, Tokyo 1010021 (JP)
(74) Representative: Beetz & Partner
(86) International application number: PCT/JP2006/319452
(87) International publication number: WO 2007/040172

(57) **Abstract**

An ultrasonic probe in which a 3-dimensional position detecting means can be attached detachably to the ultrasonic probe and operability of the ultrasonic probe does not degrade even when the position detecting means is contained in the ultrasonic probe.

An ultrasonic diagnosis apparatus employing such an ultrasonic probe is also provided.

The ultrasonic probe comprises a transducer for transmitting/receiving ultrasonic waves to/from an object to be examined, a probe head for securing the transducer, and a grip coupled on the probe head wherein the grip has a groove for detachably containing a position detecting means for detecting 3-dimensional, positional information of the ultrasonic probe.

## Description

### Technical Field

The present invention relates to a configuration of an ultrasonic probe containing means for detecting 3-dimentional positional information of the ultrasonic probe, and an ultrasonic diagnostic apparatus comprising ultrasonic probe thereof.

### Background Art

Recently, a function for improving diagnostic capability by concurrently displaying a real time ultrasonic image obtained by an ultrasonic diagnostic apparatus and a tomographic image of the same cross-section as the ultrasonic image from volume data of an obj ect to be examined being obtained in advance by an image diagnostic apparatus such as a CT diagnostic apparatus, MR diagnostic apparatus or ultrasonic diagnostic apparatus (Real time Virtual Sonography; RVS) has been in practical use so as to easily recognize the corresponding relationship between both of the images (for example, Patent Document 1).

In concrete terms, volume data of the object is acquired in advance by the image diagnostic apparatus and stored. Also, by mounting 3-dimensional position detecting means such as a magnetic sensor in an ultrasonic probe of an ultrasonic diagnostic apparatus (hereinafter arbitrarily abbreviated as a probe), capability is provided for obtaining the position of the cross-section for acquiring an ultrasonic image. Under such configuration, by obtaining the ultrasonic image in real time and detecting the 3-dimensional positional information of the ultrasonic probe, the 3-dimensional positional information of a cross-section of an ultrasonic image can be indirectly obtained. On the basis of the 3-dimensional positional information of the cross-section, the tomogrpahic image being the same as the ultrasonic image is obtained from the previously acquired volume data. Accordingly the mutual positional relationship can be easily recognized by displaying the real time ultrasonic image and the tomographic image.
Patent Document 1: JP-A-2004-89362

### Disclosure of the Invention

### Problems to be Solved

The magnetic sensor disclosed in Patent Document 1 is being provided to the ultrasonic probe, but concrete method of how to equip the sensor in the ultrasonic probe is not disclosed therein.
For example; if the position detecting means is merely attached to the probe case, it deteriorates the accuracy of positional detection by displacement of the positional detecting means whereby degrading the operability of the probe. Furthermore, in the case of transmitting the detection signals from positional detecting means to the ultrasonic diagnostic apparatus by a cable, the probe needs to be designed so that the cable will not get in the way.

Especially, problems related to the above-mentioned operability become serious in the field of puncture. Upon puncturing, an operator inserts a puncture needle accurately into the object along the puncture guide attached to the side face of the probe. In order to achieve such operation, it is necessary to improve the operability of the probe to enable the operator to perform imaging by easily and accurately positioning the probe in the vicinity of the desired region, and stably maintaining the probe at the position thereof. Therefore, position detecting means and the cable therefrom need to be placed not to get in the way of such operation. However, Patent Document 1 does not address the solution for such a problem.
Or, though it is possible to configure the probe by embedding the position detecting means in the probe case, such configuration does not allow the replacement of only the position detecting means when it breaks down. In this case, the entire probe needs to be replaced whereby forcing the operator to bear burden of expense.

The objective of the present invention is to provide an ultrasonic probe to which the position detecting means for detecting the position of the probe is detachably mounted and its operability does not degrade even when the position detecting means is contained in the probe, and an ultrasonic diagnostic apparatus comprising such ultrasonic probe.

### Means to Solve the Problem

In order to achieve the above objective, the ultrasonic probe of the present invention comprises:
a transducer for transmitting/receiving ultrasonic waves to/from an object to be examined;
a probe head for securing the transducer; and
a grip coupled on the probe head,
wherein the grip has a groove for detachably containing position detecting means for detecting 3-dimensional positional information of the ultrasonic probe.
Also, to achieve the above-mentioned objective, the ultrasonic diagnostic apparatus of the present invention comprises:
an ultrasonic probe for transmitting/receiving ultrasonic waves to/from an objective to be examined;
means for obtaining an ultrasonic image of the object from the ultrasonic signal received by the ultrasonic probe;
image recording means for recording volume data of the object obtained by an image diagnostic apparatus;
position detecting means for detecting 3-dimensional positional information of the ultrasonic probe; and
means for obtaining a tomographic image of the position corresponding to a specified cross-sectional position of the ultrasonic image from the volume data recorded in the image recording means, based on the positional information from the position detecting means,
wherein:
the ultrasonic probe comprises a transducer for transmitting/receiving ultrasonic waves to/from the object, a probe head for securing the transducer and a grip coupled on the probe head, and
the grip has a groove for detachably containing the position detecting means.

### Effect of the Invention

In accordance with the ultrasonic probe of the present invention and the ultrasonic diagnostic apparatus employing the ultrasonic probe thereof, it is possible to detachably mount in the ultrasonic probe a position detecting means for detecting the position of the probe. Operability of the ultrasonic probe can be improved instead of degraded even when the position detecting means is contained in the probe. It is also possible to provide an ultrasonic diagnostic apparatus comprising such ultrasonic probe.

### Best Mode for Carrying Out the Invention

An embodiment of the ultrasonic probe related to the present invention will be described using the diagrams. Fig. 1. is a perspective view of the left side face of an ultrasonic probe 10 related to the present embodiment, and shows a groove 32 for containing the 3-dimensional position detection means of the probe 10 (hereinafter arbitrarily abbreviated as position detecting means) and the details of a sensor cover 38. Fig. 2 is a perspective view of the right side face of the ultrasonic probe 10 related to the present embodiment, and shows a convex of a probe head 12 with respect to a grip 13, and a convex engaging part 16 to be engaged with a puncture guide 20. Fig. 3 is a front view of the ultrasonic probe 10 related to the present embodiment.

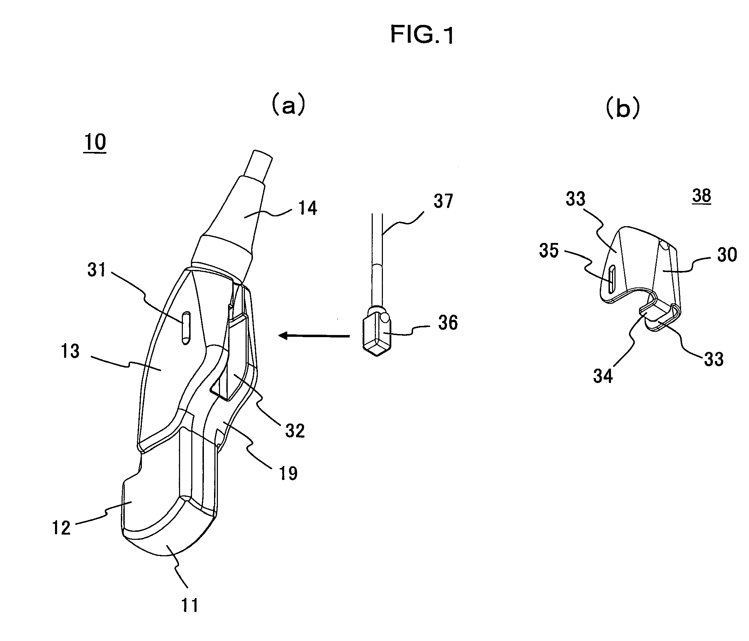

As shown in Fig. 1 ~ Fig. 3, the probe 10 has a transducer 11 for transmitting/receiving ultrasonic waves, a probe head 12 for securing the transducer and for an operator to grasp with finger tips, a grip 13 for grasping with a palm of a hand or the base between a thumb and an index finger, and a cable 14 for transmitting the ultrasonic signals obtained by transmitting/receiving ultrasonic waves to an ultrasonic diagnostic apparatus. The transducer 11 has a transducer formed by material such as piezoelectric ceramics or an ultrasonic transducer formed by a plurality of cMUT elements, and an acoustic lens for covering the ultrasonic transducer, and is arranged at the lower end of the probe head 12 in, for example, convex form. The arrangement of a transducer does not have to be limited to convex form, and may be linear, sector or other forms.

As shown in Fig. 1 (a), the groove 32 for detachably containing the position detecting means for detecting 3-dimensional positional information of the probe 10 is provided on the left side face of the grip. The position detecting means is contained in this groove 32, and a detachable sensor cover 38 is covered over the position detection means. Also, on the left side face of the probe 10, the grip 13 is smoothly connected to the probe head 12. While a magnetic sensor 36 is shown as an example of the position detecting means in Fig. 1, the position detecting means does not have to be limited to the magnetic sensor 36, and may be any device as long as it is capable of obtaining the 3-dimenisonal positional information using infra-red rays, ultrasonic waves and so on. Hereinafter, the ultrasonic probe 10 of the present embodiment will be described exemplifying the magnetic sensor 36 as the position detecting means.

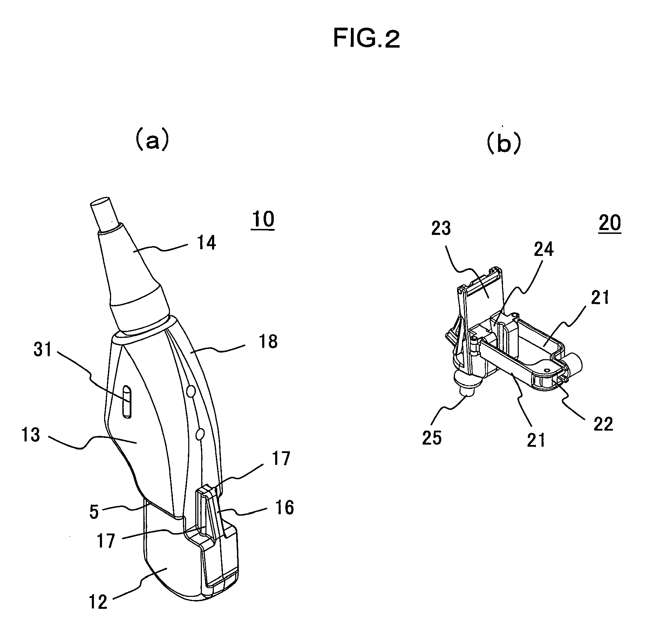

Also, as shown in Fig. 2 (a), the probe head 12 is protruded only in one longitudinal direction of the transducer 11 (that is the right side direction) with respect to the grip 13. A concave engagement unit 16 for engaging with and securing the puncture guide 20 is formed along the upper end of the protruded side of the probe head 12 to the side face of the grip 13 (that is the right side face) . On at least one side of the convex engagement unit 16, a convex portion 17 is formed in longitudinal direction of the convex engagement unit 16. The convex portion 17 is for fixing the puncture guide 20 engaged with the convex engagement 16, in longitudinal direction of the transducer 11, to keep its position from moving.

Form of the probe head 12, grip 13 and the convex engagement unit 16 in lateral direction is symmetric to the center plane parallel to the front side of the probe 10, and width of the probe head 12 in lateral direction is smaller than the width of grip 13 in lateral direction. As a result, a step portion 5 is formed between the probe head 12 and the grip 13. Also, the width of the convex engagement unit 16 in lateral direction is smaller than the width of the probe head 12 in lateral direction.

The right side plane 18 of the grip 13 is curved toward the side on which the convex engagement unit 16 is not placed (that is the left side plane 19) . And the cable 14 connected to the ultrasonic diagnostic apparatus and is for intervening transmission/reception of ultrasonic wave signals between the probe and the apparatus, is connected to the upper end of the curved grip 13. This cable 14 is pulled out in an oblique direction so as to continue with the curve of the right side plane 18 of the grip 13 with respect to the longitudinal direction of the convex engagement unit 16 (that is, in the direction of the left side place 19 and the opposite direction from the convex direction of the probe head).

The left side plane 19 of the grip 13 is concavely formed, in the plane smoothly connected from the probe head 12, curving into a shape wherein the base of an index finger and a thumb fits therein. Also, the cable 14 side of the left side plane 19 is formed in flat planar state, and the groove 32 for containing the magnet sensor is formed in the center part of the flat plane.
On the front side and the backside of the grip 13, the convex portion 31 for securing the sensor cover 38 is formed respectively. This convex portion 31 is protruded from the grip 13 in half-column shape, and extended to the same direction as the longitudinal direction of the front side and the backside. The sensor cover 38 is detachably mounted on the grip 13 by the convex portion 31 being engaged with a hole 35 of the sensor cover 38.

The magnetic sensor 36 is for detecting 3-dimensional positional information of the probe 10, and as to be described later, for indirectly obtaining the 3-dimensional positional information of the cross-section of an ultrasonic image to actualize the RVS function. Also, the cable 37 for transmitting the signals detected by the magnet sensor 36 to the main body of the ultrasonic diagnostic apparatus is placed along the probe cable 14, whereby the cable 37 for the magnet sensor 36 does not get in the way of operation of the probe 10.

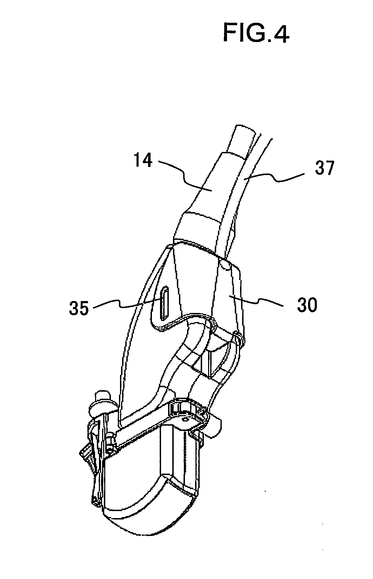

Next, the sensor cover 38 will be described. As shown in Fig. 1 (b), the sensor cover 38 has a planar basal plate 30, two engaging plates 33 formed on both ends of the planar basal plate 30 at right angle with the planar basal plate, and the square-shaped piece 34 formed from the lower end of the center part of the planar basal plate in the same direction as the engagement plates 33 in right angle with the planar basal plate 30, and these parts are constructed integrally. And the sensor cover 38 is formed in U-shape together with the planar basal plate 30 and the two engaging plates 33. In other words, the two engaging plate 33 are formed facing each other, and the width between the two engaging plates 33 is approximately the same as the width of the grip 13 in lateral direction. The width of the square-shaped piece 34 is formed to coincide with the width of the groove 32. Also, an oblong hole 35 is formed in the two engaging plates 33 respectively, and the convex portion 31 and the hole 35 are engaged, by the convex portion 31 formed on the grip 13 being inserted into the hole 35. Such configured U-shaped sensor cover 38 is attached being engaged with the grip 13 so as to cover the groove 32 containing the magnet sensor 36. Additionally, as to be described later, the convex portion 31 and the hole 35 are arranged so that the sensor cover 38 is engaged with the grip by forming a slight gap between the upper portion of the planar basal plate 30 of the sensor cover 38 and the face of the grip 13. Upon attaching the sensor cover 38 on the probe 10, the square-shaped piece 34 is slotted in the groove 32 and supports the magnet sensor 36 from underneath, whereby preventing the magnet sensor from sticking out of the groove. Fig. 4 shows the state that the sensor cover 38 is attached to the probe 10. By attaching the sensor cover 38 after the square-shaped magnet sensor 36 is contained in the groove 32, the magnet sensor 36 is stably secured without getting in the way of operation of the probe 10, whereby making it possible to improve the operability of the probe 10.

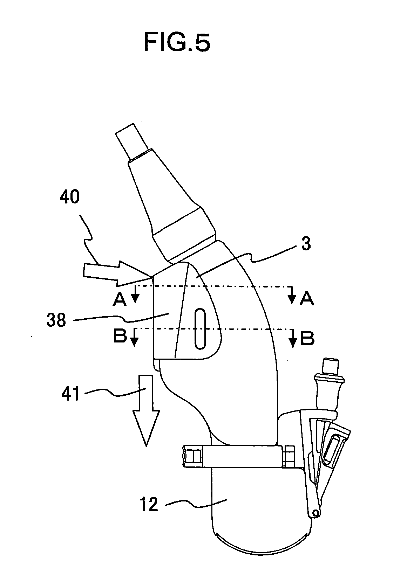

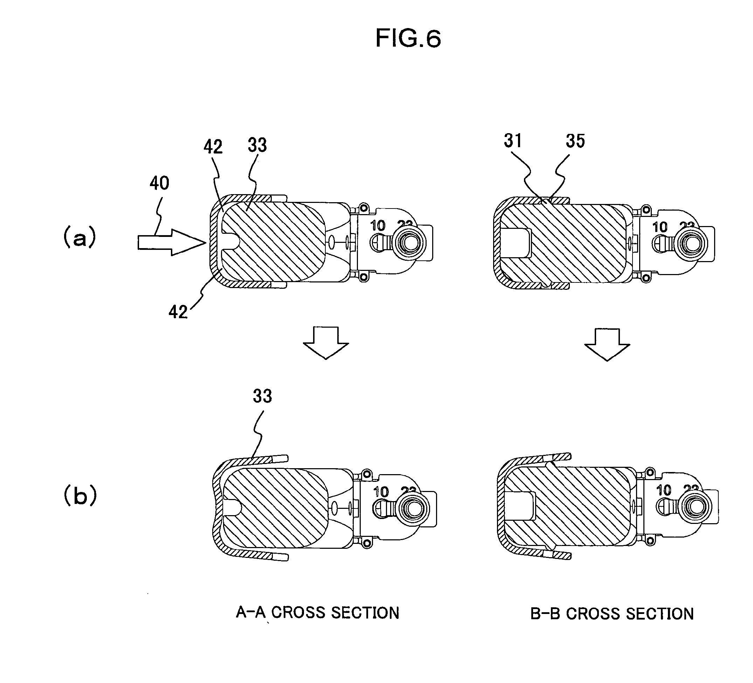

Here, the attachment/detachment of the sensor cover 38 will be described using Fig. 5 and Fig. 6. Fig. 5 shows the method upon detaching the sensor cover 38 from the probe 10, and Fig. 6 shows the cross-sections of the probe 10 in regard to the A-A plane and B-B plane illustrated in Fig. 5.
As shown in Fig. 5, when the sensor cover 38 is held down through applying force in the direction of direction 40, the force is applied in the direction of direction 41 and the sensor cover 38 comes off easily.

Fig. 6 (a) shows the state before applying force, and Fig. 6 (b) shows the state after applying force. As shown in Fig. 6 (a), the sensor cover 38 is engaged with the grip so that a slight gap 42 is formed between the upper portion of the planar basal plate 30 of the sensor cover 38 and the face of the grip 13. When the force is applied in the direction of direction 40, the planar basal plate 30 is bent toward the direction of the gap 42, and the engaging plates 33 which are configured integrally with the planar basal plate 30 are pushed out. In other words, the engaging plates 33 are stretched outward due to the planar basal plate 30 being bent. Accordingly, the hole 35 of the engaging plate 33 is detached from the convex portion 31, and the sensor cover 38 can be moved easily in the direction of direction 41. In this condition, the operator can easily remove the sensor cover 38 from the probe 10. On the other hand, the above-mentioned process will be reversed upon attachment of the sensor cover 38 on the probe 10, and the sensor cover 38 is to be stretched so that the hole 35 of the engaging plate 33 is engaged with the convex unit 31 and attached to the probe 10.
As for the attachment structure of the sensor cover 38, the structure may be used, other than the engagement of the above-mentioned hole 35 and the convex portion 31, to arrange the convex portion 31 in the rail pattern, form the rail groove on the sensor cover 38, and to attach the sensor cover 38 by sliding it on the probe 10.

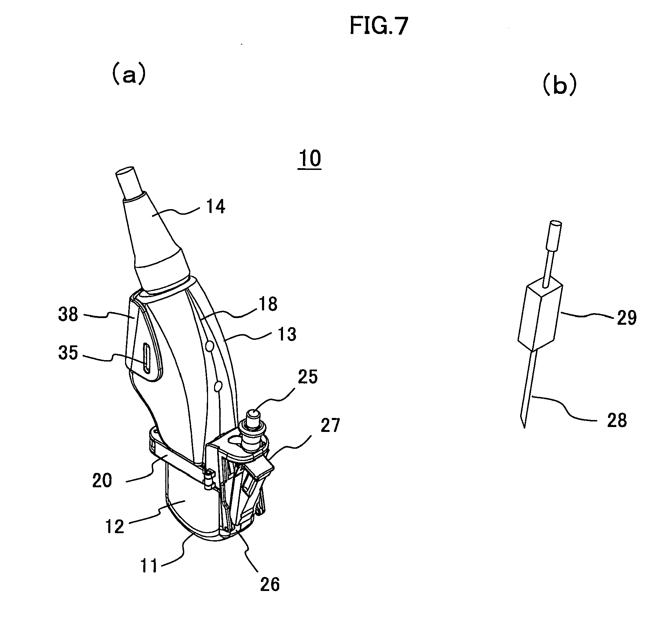

Next, in the case of performing puncture using the above-mentioned probe 10, the structure of the puncture guide 20 for guiding the puncture needle while being attached to the probe 10 on the basis of Fig. 2 and Fig. 7. Fig. 2 (b) shows a perspective view of the puncture guide 20 viewed from the side of the face being engaged with the convex engagement unit 16. Fig. 7 (a) shows a perspective view of the probe 10 attached with the puncture guide 20 and the sensor cover 38 viewed from the right side face. The puncture guide 20 is configured having a guide body 23 for securing an attachment 29 of the puncture needle 28 as shown in Fig. 7 (b), two arms 21 placed on both ends of the guide body 23, and a coupling 22 for coupling the two arms 21.

The guide body 23 is provided with a support 27 for attaching the attachment 29 for supporting the puncture needle 28. The direction of the support 27 can be varied by moving the supporting lever 25 using a supporting point 26. The support 27 can change its direction by a supporting lever 25 and a supporting point 26 . More specifically, the supporting lever 25 is provided on the upper end of the support 27, a plurality of holes for passing through and securing the supporting lever 25 to the upper end of the guide body 23 are provided, and direction of the support 27 can be changed by selecting the hole for penetrating the supporting lever 25, making the supporting point 26 as the point of support.

The concrete structure for securing the supporting lever 25 and to make it capable of changing its direction is as follows . In order to secure the supporting lever 25 in the hole for penetrating the supporting lever 25, an elastic body formed by a spring for drawing out the supporting lever 25 (not shown in the diagram) is provided on the upper end of the supporting lever 25. And the supporting lever 25 comes out of the hole by drawing out the supporting lever 25 in its axis direction by using a thumb and so on. As a result, direction of the supporting lever 25 and the support 27 can be varied because one end of the support 27 is fixed by the supporting point 26, and the supporting lever 25 and the support 27 can rotate centering on the supporting point 26. By placing the supporting lever 25 in the desired hole and canceling the draw out, the supporting lever 25 is fixed in the selected hole. Accordingly, puncture direction can be variably adjusted since the direction of the supporting lever 25 and the support 27 can be varied.
For example, in the case of inserting the puncture needle 28 at a sharp angle into an object, the supporting lever 25 is inserted and fixed into the hole on the side of grip 13. Also, in the case of inserting the puncture needle 28 at a blunt angle into the object, the supporting lever 25 is inserted and fixed into the hole farther from the grip side 13.

The arms 21 are respectively configured to rotate making a part of the side surface of the guide body 23 as a supporting point, and the two arms are coupled by the coupling unit 22. The coupling unit 22 is secured by the tightening of a spring. The spring of the coupling unit 22 is provided on one of the two arms 21, and the U-shaped engaging part of the coupling unit 22 is provided on the other arm 21. The spring can be rotated by rounding on the arm 21, and is to be engaged with the U-shaped engaging part by rotating the spring upon fixing the spring on the U-shaped engaging part. The puncture guide 20 is formed in a box shape by the guide body 23, the two arms 21 and the coupling unit 22, due to the coupling of the two arms with the engaging part 22. This engaging part 22 may be configured so that the arms 21 are to be secured in a set-in style.

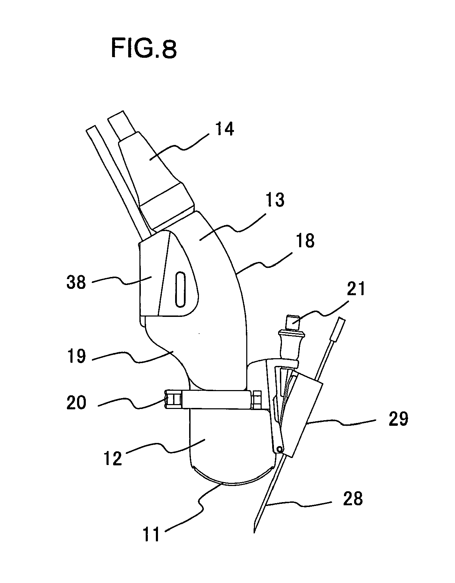

Also, the concave engaging part 24 is formed on the guide body 23, and the concave engaging part 24 is formed to be engaged with the convex engaging part 16 and its convex portion 17. Upon the puncture guide 20 being attached to the probe 10, the concave engaging part 24 is inserted in the longitudinal direction of the convex engaging part 16 employing the convex engaging part 16 as a guide.
Then after the puncture guide 20 is engaged with the convex engaging part 16 and the convex portion 17, the puncture guide 20 is secured to the probe 10 by the two arms 21 being engaged with the probe head 12 by surrounding its peripheral surface. In this regard, by the end face of the two arms 21 being held down by the step portion 5 between the probe head 12 and the grip 13, the puncture guide 20 is secured in the longitudinal direction of the probe. In this way, the puncture guide 20 is secured by the convex engaging part 16, the convex portion 17, and the step portion 5 between the probe head 12 and the grip 13 in any direction of the vertical, lateral or front side-backside of the probe 10. By such puncture guide 20 being firmly secured to the probe 10, the direction of the puncture needle can be stabilized. Fig. 8 is a front view of the probe 10 on which the puncture guide 20 is attached.

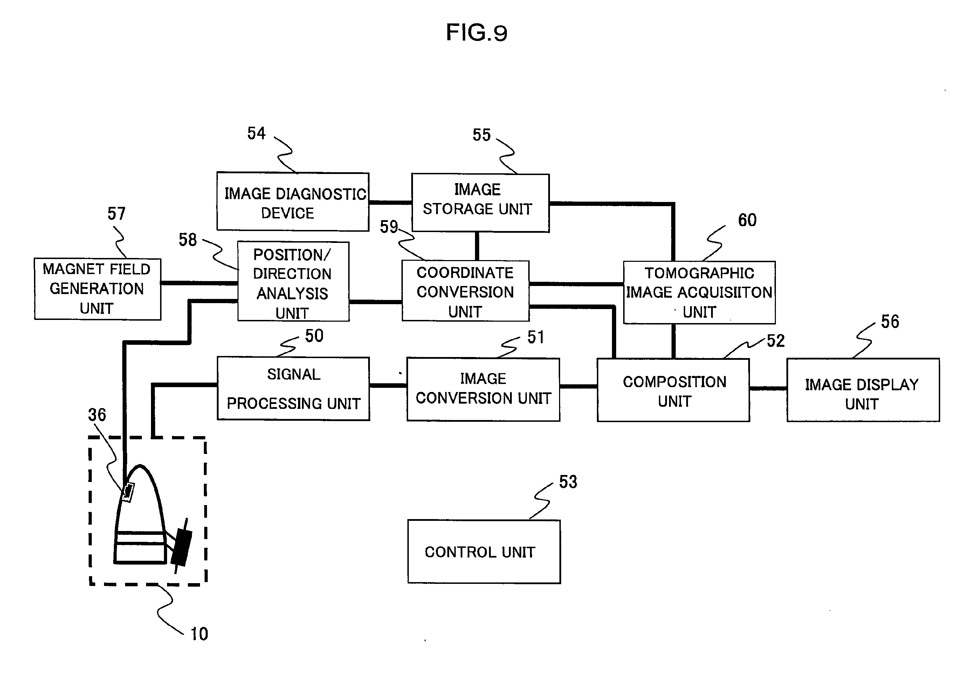

Next, the ultrasonic diagnostic apparatus comprising the above-described ultrasonic probe will be described. Fig. 9 is a block diagram showing the general configuration of the ultrasonic diagnostic apparatus. The ultrasonic diagnostic apparatus has an ultrasonic probe 10, a signal processing unit 50 connected with the ultrasonic probe 10, an image conversion unit 51 connected with the signal processing unit 50, a composition unit 52 connected with the image conversion unit 51, an image display unit 56 connected with the composition unit 52, and a control unit 53 connected with the respective units.
Moreover, in order to actualize the RVS function, the ultrasonic diagnostic apparatus further comprises an image recording unit 55 for recording the volume data of the object obtained by any image diagnostic apparatus 54 of the CT diagnostic apparatus, MR diagnostic apparatus or the ultrasonic diagnostic apparatus, a magnet generation unit 57, a magnet sensor 36 for detecting 3-dimensional positional information of the ultrasonic probe 10, a position/direction analyzing unit 58 connected with the magnet sensor 36 and the magnet generation unit 57, a coordinate conversion unit 59 connected with the position/direction analyzing unit 58 and the image recording unit 55, and a tomographic image acquisition unit 60 connected with the coordinate conversion unit 59 and the image recording unit 55. The composition unit 52 is connected also with the coordinate conversion unit 59.

The ultrasonic probe 10, as described above, has the groove 32 for containing the magnet sensor 36 in the grip 13 for grasping the probe 10, and comprises convex engaging part 16 for engaging and attaching the puncture guide 20 to the probe head 12 which is for transmitting/receiving ultrasonic waves to/from the object.
The signal processing 50 is for signal processing such as amplifying and phasing the receiving signals received from the ultrasonic probe 10.
The image conversion unit 51 is for converting the receiving signals outputted from the signal processing unit 50 into an ultrasonic image, and referred to as so-called digital scan converter.
The composition unit 52 is for generating an image representing at least one of the ultrasonic image converted by the image conversion unit 51 and the tomographic image acquired by the tomographic image acquisition unit 60.
The image display unit 56 displays the image generated in the composition unit 52.
The control unit 53 is a CPU for controlling the above-described respective units, and the connecting lines for controlling the above-described respective units are omitted in Fig. 9.
The magnet sensor 36 is a receiver for detecting magnet field of a triaxial orthogonal system, and the magnet generating unit 57 for generating the magnet field of the triaxial orthogonal system is provided at the bedside. The 3-dimensional position and the direction of the probe 10 is indirectly obtained, by the magnet sensor 36 being a receiver indirectly detecting the position and direction in the 3-dimensional space of the receiver in the 3-dimensional coordinate space in the magnet field space set by the magnet generating unit 57.

In the case of performing ultrasonic image diagnosis, the operator starts transmission/reception of ultrasonic wave signals by applying the ultrasonic probe 10 on the object and holding down a transmission switch (not shown in the diagram) . The signal processing unit 50 performs the signal processing such as amplifying and phasing the receiving signals received from the ultrasonic probe 10. The image conversion unit 51 converts the receiving signals outputted from the signal processing unit 50 into an ultrasonic image. The image display unit 56 displays the ultrasonic image converted by the image conversion unit 51. In the case of performing puncture, the operator finds a diseased part of the obj ect such as cancer cells using the displayed ultrasonic image, and punctures the puncture needle 28 into the affected area from the body surface of the object. Upon puncturing, the operator adjusts the position of the inserting/pulling direction of the puncture needle 28 according to the depth of the diseased part from the body surface of the object as previously described.

Furthermore, the case of performing ultrasonic image diagnosis and puncturing in conjunction with the RVS function will be described. In RVS function, in order to obtain the 3-dimensional positional information of the ultrasonic image being obtained in real time, the position/direction analyzing unit 58 generates a magnetic field to the magnetic field generating unit 57, and the 3-dimensional position and the direction of the magnetic sensor 36 that is the ultrasonic probe 10 based on the magnetic generating unit 57 by analyzing the signals detected by the magnetic sensor 36. Next, the coordinate conversion unit 59 obtains the 3-directional position or the direction of the cross-section of the ultrasonic image from the 3-dimensional position or the direction of the ultrasonic probe 10 analyzed in the position/direction analyzing unit 58, then obtains the 3-dimensional position of the cross section of the volume data corresponding to the cross-section of the ultrasonic image. Next, the tomographic image acquisition unit 60 reconstructs a tomographic image of the cross-sectional position of the volume data converted by the coordinate conversion unit 59 from the volume data. Accordingly, the tomographic image of the same cross-section as the ultrasonic image being obtained in real time can be obtained as a reference image. Lastly, at least one of the above-described real time ultrasonic images and/or the reference images, preferably the two images being juxtaposed, is displayed on image display unit 56.

As described above, upon displaying the ultrasonic image of the specified cross-sectional position of the object, the tomographic image of the position corresponding to the specified cross-sectional position of the ultrasonic image is obtained from the volume data recorded in the image recording unit 55, using the output of the magnetic sensor 36 contained in the ultrasonic probe 10.

As a preparation for implementing the RVS function, the following steps are to be proceeded beforehand. First, the imaging of the object is performed by the image diagnostic apparatus 54 such as CT diagnostic apparatus, MR diagnostic apparatus or ultrasonic diagnostic apparatus, and the obtained volume data is stored in the image recording unit 55. Next, the reference coordinate system of the volume data and the reference coordinate system of the object being imaged by the ultrasonic diagnostic apparatus are corresponded to the standard coordinate system that is the common coordinate system. For that purpose, the operator sets the reference point on the reference image that is reconstructed based on the previously acquired volume data. The operator then sets the reference point of the object by coordinating the position of the ultrasonic probe 10 with the position of the object corresponding to the previously set reference point. The position of the ultrasonic probe 10 is detected by the magnet sensor, and the reference point on the volume data and the reference point on the object are corresponded to each other. In this way, the reference coordinate system of the object is corresponded to the standard coordinate system, and the coordinate system correspondence data for corresponding the reference coordinate system of the volume data to the standard coordinate system is created and stored. At the time of ultrasonic diagnosis after these steps, the position and the direction of the imaging cross-section is obtained based on the 3-dimensional position and the direction of the ultrasonic probe detected by the magnetic sensor, and the reference image corresponding to this cross section is extracted from the volume data and displayed along with the ultrasonic image.

Next, the puncture in conjunction with the RVS function, performed by the above-mentioned ultrasonic diagnostic image comprising the ultrasonic probe 10 shown in Fig. 8, wherein the first magnetic sensor 36 is contained in the grip 13, the sensor cover 38 is attached, and the puncture guide 20 is attached to the probe head 12, will be described. Upon puncturing, the puncture attachment 29 in which the puncture needle 28 is mounted is attached to the puncture guide 20.

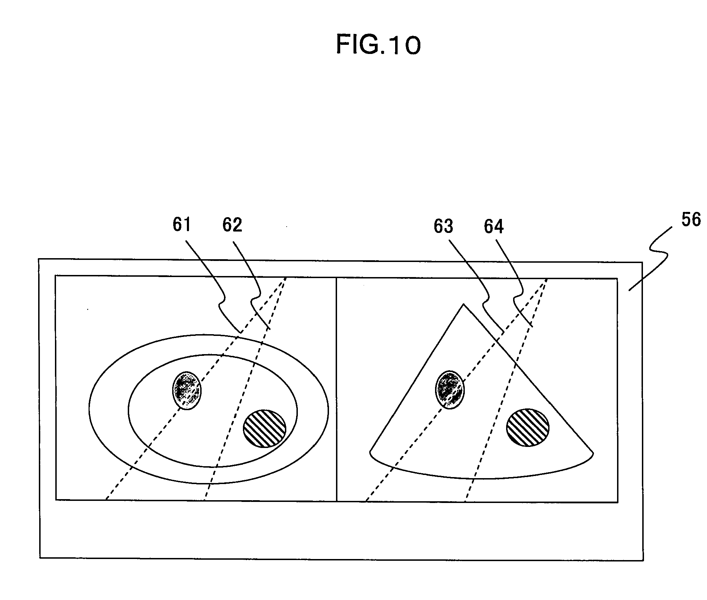

Fig. 10 shows a display pattern wherein the ultrasonic image and the tomographic image obtained by the RVS function, using the probe 10 as shown in Fig. 8 are displayed on the image display unit 56. The image on the left is a tomographic image obtained from volume data of the image diagnostic apparatus 54, and the image on the right is an ultrasonic diagnostic image obtained in real time.
In the ultrasonic image, a dashed line 63 is a guide line showing the puncture direction in the case that the puncture needle 28 is inserted at an obtuse angle, and a dashed line 64 is a guide line showing the punctured direction in the case that the puncture needle 28 is inserted at a sharp angle. As described above, the holes are provided in two places for securing the supporting lever 25 to the guide body 23, and it is designed that any of those holes are used for securing the supporting lever 25. These two guidelines can be displayed at the position corresponding to the direction of the puncture needle 28 being set by the position of the holes and the supporting lever 25. The guideline is displayed at the same place on the tomographic image obtained from the volume data so as to correspond to the position of the two guidelines on the ultrasonic image. A dashed line 61 is a guideline corresponding to the position of the dashed line 63, and a dashed line 62 is a guideline corresponding to the position of the dashed line 64. Therefore, by displaying the guidelines on the two different images of the ultrasonic image and the tomogrpahic image, the operator can easily specify the position of the diseased area, whereby making it possible to easily draw up a puncture plan and to perform puncture effectively and accurately.

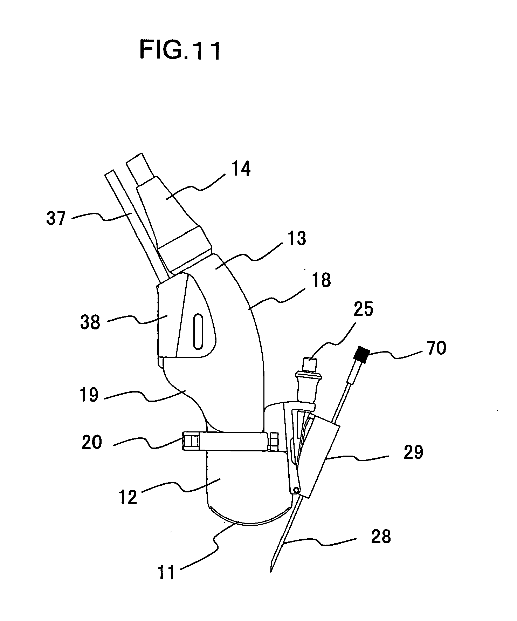

Further, an example for indicating the front edge position of the puncture needle on the guideline will be described. For that purpose, as shown in Fig. 11, a second magnetic sensor 70 is placed at the edge of the puncture needle 28. The second magnetic sensor is mounted with the above-mentioned receiver for detecting the magnetic field of a triaxial orthogonal system, in the same manner as the first magnetic sensor 36. The second magnetic sensor is also connected to the position/direction analyzing unit 58, and the signals from the second magnet sensor are analyzed in the same manner as the first magnet sensor 36.

First, the method for specifying the 3-dimenisonal position of the second magnetic sensor 70 with respect to the first magnet sensor 36 to be set in the sensor cover 38 will be described. After the second magnetic sensor 70 is fixed on the front edge of the puncture needle 28, the operator places the second magnetic sensor 70 at the position adjacent to the attachment 29. Then the original point position of the second magnetic sensor 70 with respect to the first magnetic sensor 36 set in the sensor cover 38 is specified, and the front edge position (needlepoint) of the puncture needle 28 is specified from the position, angle and length of the puncture needle 28 of the second magnetic sensor 70. In other words, the original point of the second magnetic sensor 70 is specified, and the position from the original point to the needlepoint is specified. Since the position of the second magnetic sensor 70 moves as the same distance as the distance that the puncture needle 28 moved, the moving distance of the puncture needle 28 can be specified. Also, the puncture needle 28 can move only in a 1-dimenisonal direction due to the attachment 29, the moving direction of the puncture needle is also specified.

The position/direction analyzing unit 58 and the coordinate conversion unit 59 specifies the guideline of the puncture needle 28 and the position of the needlepoint by converting the moving distance and the moving direction of the puncture needle 28 into the 3-dimensional positional coordinate based on the detected signals from the first magnet sensor 36 and the second magnet sensor 70. Then the coordinate convers ion unit 59 transmits the guideline of the puncture needle 28 and the positional information of the needlepoint to the composition unit 52. The composition unit 52 displays the guideline of the puncture needle and the position of the needlepoint on the image display unit 56 based on the received positional information.

The guidelines are displayed as shown in Fig. 10. The dashed line 62 is the guideline showing the puncture direction in the case of inserting the puncture needle 28 at an obtuse angle, and the dashed line 63 is the guideline showing the puncture direction in the case of inserting the puncture needle 28 at a sharp angle. The angle of the puncture needle 28 to be set is selected by the position to apply the second magnetic sensor 70 to the attachment 29, that is the original point position. Accordingly, since the setting condition of the angle of the puncture needle 28 is specified by the original position information, only the selected puncture direction or the two puncture directions can be displayed on the display screen. In the case of displaying the two puncture directions, the display pattern may be differentiated between the selected puncture direction and the other puncture direction.

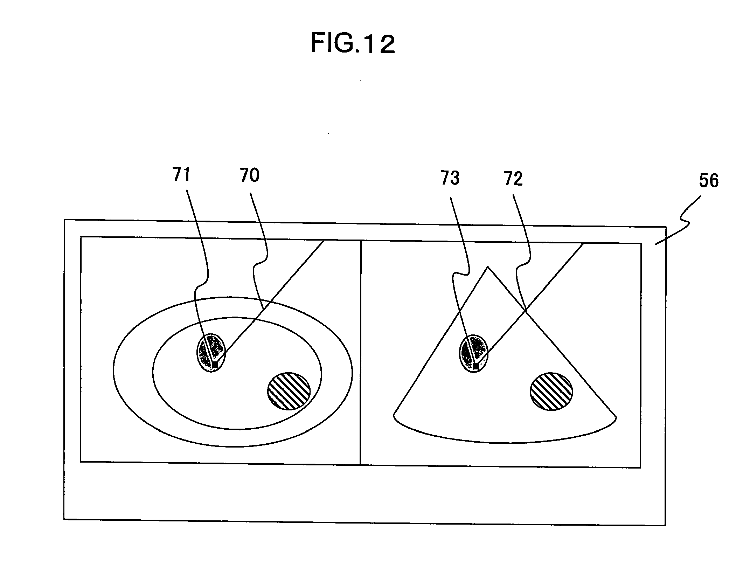

The guideline of the puncture needle 28 and the position of the needlepoint are displayed as shown in Fig. 12. The image on the left is a tomographic image obtained from the volume data of the image diagnostic apparatus 54, and the image on the right is an ultrasonic image. In the ultrasonic image on the right, a solid line 72 indicates a main body of the puncture needle in the case of inserting the puncture needle 28, and a dot 73 indicates a needlepoint of the puncture needle 28. In the tomographic image on the left, a solid line 70 indicates the main body of the puncture needle in the case of inserting the puncture needle 28, and a dot 71 indicates the needlepoint of the puncture needle 28. By specifying the front edge position (needlepoint) of the puncture needle 28 from the position and angle of the second magnetic sensor 70 and the length of the puncture needle 28 and displaying the needlepoint of the puncture needle 28, the operator can cognize the transition of the puncture needle in real time. Therefore, since the puncture needle 28 and the needlepoint are displayed on the two different images of the ultrasonic image and the tomogrpahic image, the operator can perform the puncture treatment safely.

The guideline such as the one shown in Fig. 10 may be displayed on the image shown in Fig. 12. Also, while the second magnetic sensor 70 is used for the receiver for detecting a magnetic field of a orthogonal system, any device can be used as long as it can specify the position with respect to the first magnetic sensor 36 set in the sensor cover 38.

Above is the description of the probe 10 related to the present embodiment and the ultrasonic diagnostic apparatus comprising the probe, and characteristic configuration of the ultrasonic probe 10 can be put together as follows. The magnetic sensor 36 is provided on the left side face of the grip 13. The probe head 12 is protruded on one end of the transducer 11 in longitudinal direction with respect to the grip 13. The convex engaging part 16 for engaging and securing the puncture guide 20 is formed along the protruded side of the probe head 12 from the upper end on the convex side to the side face (right side face) of the grip 13. The right side face 18 of the grip 13 is curved toward the side to which the convex engaging part 16 is not provided (left side face). In other words, the shape of the ultrasonic probe 10 is formed asymmetric on the left and right sides, and the grip 18 and the draw-out direction of the cable 14 are formed to be away from the side that guides the puncture needle 28. Due to the above-described characteristic configuration, detecting means for detecting 3-dimensional position of the probe 10 can be detachably attached to the probe 10. Also, operability of the probe 10 does not degrade even when the position detecting means is contained in the probe 10. Since the space on the side that guides the puncture needle 28 is widened, operability of the probe 10 can be improved. Further, safety of the puncture treatment can be improved by displaying the guideline of the puncture needle 28 or needlepoint on the screen through comprising magnet sensors 36 and 70 and using them together with RVS function.

### Brief Description of the Diagrams

Fig. 1 shows a perspective view of the left side face of an ultrasonic probe, a magnet sensor and a sensor cover related to the first embodiment of the present invention.
Fig. 2 shows a perspective view of the right side face of an ultrasonic probe and a puncture guide related to the first embodiment of the present invention.

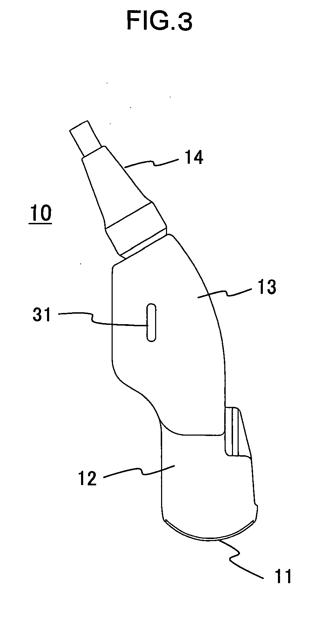

Fig. 3 shows a front view of an ultrasonic probe related to the first embodiment of the present invention.
Fig. 4 shows a state that a sensor cover is attached to the ultrasonic probe related to the first embodiment of the present invention.
Fig. 5 shows a method for detaching the sensor cover from the ultrasonic probe related to the first embodiment of the present invention.
Fig. 6 shows a cross-sectional view of the ultrasonic probe in regard to the A-A plane and B-B plane shown in Fig. 5.
Fig. 7 shows a perspective view of the right side face illustrating a state that a puncture guide and the sensor cover are attached to the ultrasonic probe related to the first embodiment of the present invention.
Fig. 8 shows a front view illustrating a state that the puncture guide is attached to the ultrasonic probe related to the first embodiment of the present invention.
Fig. 9 shows a block configuration of an ultrasonic diagnostic apparatus related to the first embodiment of the present invention.
Fig. 10 shows a display pattern of a puncture guideline displayed on an image display unit.
Fig. 11 shows a configuration wherein a second magnetic sensor is provided on the forefront of a puncture needle.
Fig. 12 shows a display pattern for displaying a needlepoint of a puncture needle on an image display unit.

### Description of the Symbols

10... ultrasonic probe, 11... transducer, 12... probe head, 13... grip, 14... cable, 20... puncture guide, 28... puncture needle, 38... sensor cover, 61,62,63 and 64... puncture guideline, 70... second magnetic sensor, 71... needlepoint, 73... needlepoint.

## Claims

1. An ultrasonic probe comprising:
a transducer for transmitting/receiving ultrasonic waves to/from an object to be examined;
a probe head for securing the transducer; and
a grip to be coupled with the probe head,
wherein the grip has a groove for detachably containing a first position detecting means for detecting 3-dimensional positional information of the ultrasonic probe.

2. The ultrasonic probe according to claim 1, **characterized in that** the probe head is protruded in longitudinal direction of the transducer toward the side without the groove opposed to the side having the grip.

3. The ultrasonic probe according to claim 2, **characterized in that** the side having the groove of the probe head is smoothly coupled on the grip.

4. The ultrasonic probe according to claim 2, **characterized in that** the width of the probe head in lateral direction is formed narrower than the width of the grip in lateral direction, and a step is provided at the border between the probe head and the grip.

5. The ultrasonic probe according to claim 2, **characterized in that** the surface of the protruded direction side of the grip is curved toward the opposite direction to the protruded direction.

6. The ultrasonic probe according to claim 5, wherein the cable connected to the grip is to be drawn out in the opposite direction to the protruded direction of the probe head.

7. The ultrasonic probe according to claim 2, **characterized in that** a detachable cover for covering the first position detecting means contained in the groove is attached to the grip.

8. The ultrasonic probe according to claim 7, wherein:
the cover has a planar basal plate which covers the first position detecting means contained in the groove and engaging plates to be engaged with the grip on both ends of the planar basal plate, and
the grip has a cover engaging part to be engaged with the engaging plates.

9. The ultrasonic probe according to claim 8, wherein:
the cover is engaged to the grip so that a gap is formed' between the planar basal plate and the grip, and
the engaging plates and the cover engaging part are engaged so that they are detached from each other due to the expanse of the engaging plates based on the deformation caused by the planar basal plate being pressed down.

10. The ultrasonic probe according to claim 7, **characterized in that** a convex engaging part to be engaged with a puncture guide for holding the puncture guide is provided at the upper end of the probe head on the side in the protruded direction.

11. The ultrasonic probe according to claim 10, **characterized in that** a convex portion for securing the puncture guide in the longitudinal direction of the transducer is formed on at least one side face of the convex engaging part.

12. The ultrasonic probe according to claim 11, wherein the puncture guide has a concaved engaging part to be engaged with the convex engaging part and the convex portion of the probe head.

13. The ultrasonic probe according to claim 12, wherein the puncture guide has an arm portion for surrounding the probe head and securing the puncture guide on the probe head, **characterized in** being engaged on the convex engaging part, and that the arm portion surrounding the probe head is secured in the longitudinal direction of the probe due to being held by a step between the probe head and the grip.

14. The ultrasonic probe according to claim 10, wherein the puncture guide comprises a support for detachably mounting an attachment for supporting a puncture needle.

15. The ultrasonic probe according to claim 14, wherein the puncture guide is provided with a plurality of holes for variably securing the angle for configuring a supporting lever for changing the direction of the support.

16. An ultrasonic diagnostic apparatus comprising:
an ultrasonic probe for transmitting/receiving ultrasonic waves to/from an object to be examined;
means for acquiring an ultrasonic image of the object from the ultrasonic signals received by the ultrasonic probe;
image storing means for storing volume data of the object acquired by the image diagnostic apparatus;
a first position detecting means for detecting 3-dimensional positional information of the ultrasonic probe; and
means for acquiring a tomographic image of the position corresponding to a specified cross-sectional position of the ultrasonic image based on the positional information from the first position detecting means,
wherein:
the ultrasonic probe comprises a transducer for transmitting/receiving ultrasonic waves to/from the object, a probe head for securing the transducer, and a grip to be coupled on the probe head, and
the grip has a groove for detachably containing the first position detecting means.

17. The ultrasonic diagnostic apparatus according to claim 16, **characterized in** comprising a second position detecting means in a puncture needle to be inserted into the object by guidance of the puncture guide attached to the ultrasonic probe.

18. The ultrasonic diagnostic apparatus according to claim 16, **characterized in** comprising display means for displaying at least one of the ultrasonic image and the tomographic image, wherein the display means displays a guideline indicating the transit position of the puncture needle superimposing onto at least one of the ultrasonic image and the tomographic image following the movement of the puncture needle based on the positional information from the first position detecting means and the second position detecting means.

19. The ultrasonic diagnostic apparatus according to claim 18, wherein the display means displays the needlepoint position of the puncture needle following the movement of the puncture needle, superimposing onto at least one of the ultrasonic image and the tomogrpahic image.

20. The ultrasonic diagnostic apparatus according to claim 18, **characterized in** comprising magnetic field generating means that varies in triaxial orthogonal directions, wherein the first position detecting means and the second position detecting means are a magnetic sensor for detecting the 3-dimensional position.
